# EUROPEAN PATENT SPECIFICATION

(11) **EP 1 838 731 B1**
(45) Date of publication and mention of the grant of the patent: **07.11.2012**
(21) Application number: 05729938.0
(22) Date of filing: 23.03.2005
(51) Int. Cl.: C07K 16/00, A61K 39/395, A61P 31/04

(54) **USE OF FERROUS IONS FOR INCREASING THE IMMUNOREACTIVITY OF IMMUNOGLOBULIN PREPARATIONS**
VERWENDUNG VON EISEN(II)-IONEN ZUR VERBESSERUNG DER IMMUNREAKTIVITÄT VON IMMUNGLOBULINZUBEREITUNGEN
UTILISATION D'IONS FERREUX POUR AUGMENTER L'IMMUNOREACTIVITE DE PREPARATIONS D'IMMUNOGLOBULINE

(30) Priority: 23.11.2004 BG 10894504
(43) Date of publication of application: 03.10.2007
(73) Proprietor: Vassilev, Tchavdar L., 1618 Sofia (BG)
(72) Inventor: VASSILEV, Tchavdar, L., 1618 Sofia (BG); DIMITROV, Jordan, D., 1233 Sofia (BG)
(74) Representative: Stefanova, Stanislava Hristova
(86) International application number: PCT/BG2005/000003
(87) International publication number: WO 2006/056031

(56) References cited:
- WO-A-01/44298
- US-B1- 6 465 511
- BOUVET J P ET AL: "DRAMATIC INCREASE IN NATURAL AUTOANTIBODY ACTIVITY FOLLOWING TREATMENT OF NORMAL HUMAN IMMUNOGLOBULIN WITH DISSOCIATING AGENTS" FASEB JOURNAL, FED. OF AMERICAN SOC. FOR EXPERIMENTAL BIOLOGY, BETHESDA, MD, US, vol. 14, no. 6, 20 April 2000 (2000-04-20), page A1131, XP000992959 ISSN: 0892-6638
- NORDSTRÖM, D. ET AL RHEUMATOL. INT. no. 17, 1997, pages 67 - 73
- DA SILVA, A.D. ET AL INFLAMMATORY BOWEL DISEASES no. 9, 2003, pages 316 - 320

## Description

### Field of invention

The invention relates to the use of ferrous (Fe(II)) ions for increasing the immunoreacivity of immunoglobulin preparations, especially for increasing their binding activity to foreign and self antigens, their anti-idiotypic activity and anti-inflammatory properties.

### Background of the invention

Natural IgG antibodies to self- and to foreign antigens are known to be a part of the normal antibody repertoire (1, 2). These natural antibodies end up in the therapeutic immunoglobulin (IVIg) preparations for intravenous administration that are produced from pooled plasmas from healthy donors. IVIg preparations are used for the treatment of a variety of systemic and organ-specific autoimmune and inflammatory diseases (3). It has been suggested that the natural IgG autoantibodies present in IVIg are responsible for at least a part of the beneficial therapeutic effect observed in the treated patients (4-6).

Immunoglobulin preparations contain anti-idiotypic antibodies to idiotypes of disease-associated autoantibodies and neutralize the latter (7-9).

Patent application PCT/US95/14869 describes a method for enriching the immunoglobulin preparations with antibodies to antigen receptors of T lymphocytes. This method is based on the use of a fraction of an immunoglobulin preparation obtained by elution from an immunoaffinity column containing an immobilized synthetic peptide from the mentioned antigen receptor.

A method for treatment of systemic lupus erythematosus in animals was published that uses a fraction of a pooled immunoglobulin preparation enriched in antibodies to idiotypic determinants of anti-DNA antibodies from lupus patients. These anti-idiotypic antibodies were obtained by immunoaffinity chromatography of Mg on immobilized mixture of anti- DNA antibodies from 55 lupus patients (10).

A method for augmenting of immunoreactivity of immunoglobulin preparations is published (11) that is based on the following procedure. The immunoglobulin solution is passed through a gel-filtration column. This results in the subsequent elution of the dimeric and of the monomeric fractions. The dimeric fraction obtained is enriched in natural autoantibodies.

Dietrich et al. 1992 have published a method for enriching of immunoglobulin preparations with natural autoantibodies and with anti- idiotypic antibodies that is based on the isolation of the immunoglobulin fraction that binds to immobilized F(ab')₂ fragments of IVIg (12).

Patent application BG108223 describes a method for increasing the autoreactivity of therapeutic immunoglobulin solutions by treatment in a static magnetic field.

The effect of all methods described above on the immunoreactivity of immunoglobulin preparations is, however, weak.

Patent EP1237928 describes a method for enhancing the activity of an immunoglobulin preparation by treating it at low pH, high pH or with chaotropic agents (urea, guanidine and guanidinium salts or thiocyanates). It remains, however, unclear whether this treatment results in the appearance of new biologically relevant beneficial characteristics of the treated preparation.

The biological effects of ferrous (Fe(II)) ions on rat lymphocytes is studied in *in vitro* experiments (13). No toxic effects were found, but this treatment applied simultaneously with a constant magnetic field resulted in the death of the cells. It is known that ferrous ions can potentiate the generation of free radicals in certain biological systems that results in the suppression of bacterial growth (14).

Patent. US-A-6465511 describes treating septic shock using a transition metal complex, which includes transition metals as iron, copper, zinc, manganese, nickel and suitable chelating agents. This transition metal complex may be administered alone or with a NO synthetase inhibitor and acts by scavenging nitric oxide (NO) in the body. The cited invention is solely based on the suppressive effect on NO levels and is directed to the treatment of hypotension in septic shock in which an excess of NO contributes to the pathophysiology of the condition.

It is known from Nordström, D. et al (1997), Rheumatol. Int. 17:67-73 that oral iron supplementation is used during investigation of the response to recombinant human erythropoietin (r-HuEPO) when treating anemia of chronic disease (ACD) in patients with rheumatoid arthritis (RA). The original study protocol of treatment regimen included monitoring of the response to the r-HuEPO given as subcutaneous injection and only for patients who were iron-deficient- in combination with oral iron supplementation. The latter is given with the aim of elevating the haemoglobin levels in the patient's blood. The authors do not investigate the cytokines' behaviour in the EPO response, associated with RA.

Subject matter of publication of de Silva, A. D. et al (2003), Inflammatory Bowel Diseases (IBD) 9:316-320 is to retrospectively compare usage, tolerance and efficacy of oral iron therapy in patients with IBD and noninflamatory causes of iron deficiency. Iron therapy is discussed as an often used without a formal diagnosis of iron deficiency having been made; the publication does not suggest treatment of conditions caused by the uncontrolled production of inflammatory cytokines.

No data on the ability of ferrous ions to affect the immunoreactivity and anti-inflammatory activity of immunoglobulin preparations have been reported so far.

### Description

The present invention relates to the use of ferrous ions aiming to increase the immunoreacivity of immunoglobulin preparations, especially for increasing their binding activity to foreign and to self antigens, their anti-idiotypic activity and anti-inflammatory properties. In particular, the invention relates to the use of an agent to increase immunoreactivity of immunoglobulin preparations, the agent represents ferrous (Fe(II)) ions in the form of a ferrous salt, added to a single dose of an immunoglobulin preparation in a final concentration from 0.5 to 2 mM.

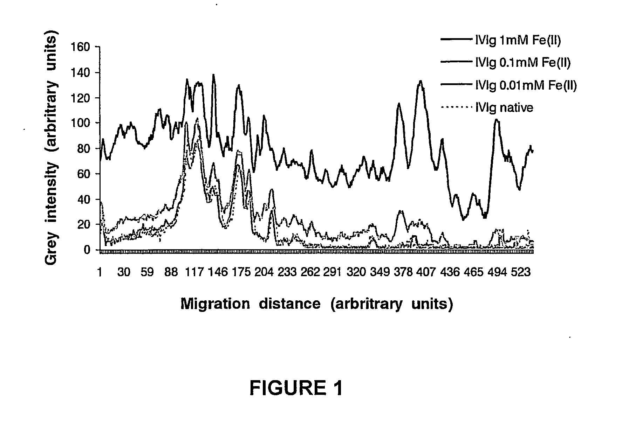

The unexpected new property of the ferrous ions according to the invention, to augment the anti-inflammatory activity of immunoglobulin preparations, is confirmed by the results from *in vitro* as well as from *in vivo* experiments (Figs. 1-11). All data obtained prove the effects of the Fe(II)-treatment on the immunoreactivity, on the anti-idiotypic activity and on the anti-inflammatory properties of the therapeutic immunoglobulin preparations. The contact of the preparation with Fe(II) ions was shown to result in increased binding to the pro-inflammatory cytokine hIFN-γ and in increased survival of animals with septic shock.

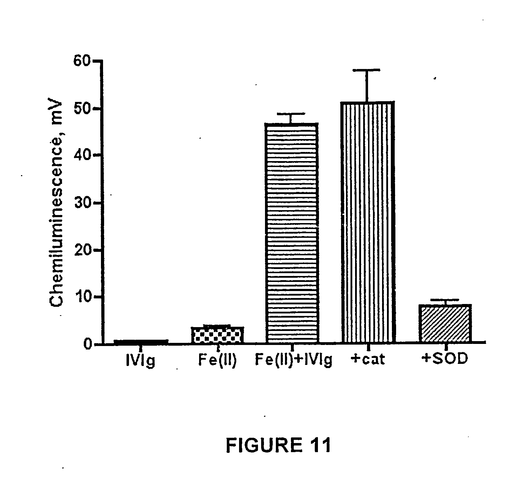

To get a better understanding of the mechanisms of action of ferrous ions on the therapeutic immunoglobulin preparations chemiluminescence analyses were performed. The signal was weak (0.5 mV) in the control Mg solution. The addition, however, of 0.5 mM ferrous ions increased sharply the strength of the signal (to 20 mV) (Fig. 11). This signal was suppressed in the presence of superoxide dismutase, but not of catalase, an observation that pointed out that superoxide anion was generated in the system. This radical could convert via a series of reactions into still more aggressive reactive oxygen species (singlet oxygene and hydroxyl radical) that have the ability to introduce changes into macromolecules.

The structural modifications in the immunoglobulin molecules resulting from the Fe(II)-treatment were studied using spectrofluorimetry. A decrease in the strength of the fluorescence signal and displacement of the emission maxima were observed. These observations suggest that irreversible changes involving tryptophan residues have taken place resulting in alteration of the primary and tertiary protein structure.

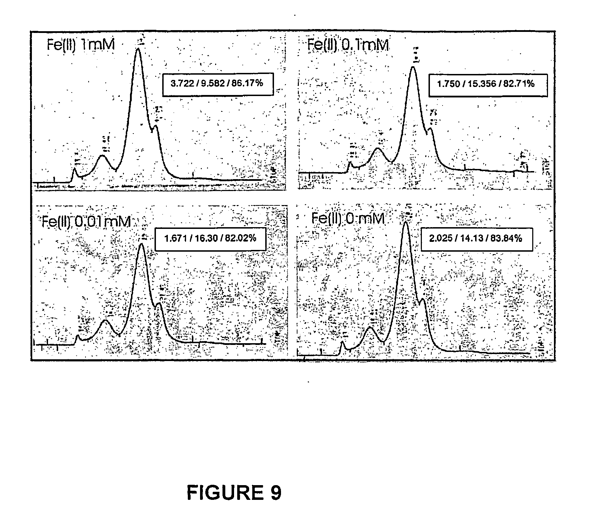

The novel application for ferrous ions, according to the invention, has the advantage that it does not induce gross changes in the immunoglobulin molecules. The ability of native and of Fe(II)-treated Mg to fix complement were compared using the method required by the European Pharmacopoeia (15). No differences in the anti-complementary activity were detected. The HPLC curves of native and of treated with 0.01, 0.1 and 1 mM ferrous ions Mg were also compared. The content of monomeric, dimeric and polymeric immunoglobulin molecules was comparable (Fig. 9). This shows that the immunoglobulin preparations treated according to this invention meet the requirements for therapeutic immunoglobulin solutions and could be administered by the intravenous route.

### Brief description of the Figures

Figure 1 presents the increased binding of a Fe(II) ions-treated immunoglobulin preparation for intravenous administration to E. coli antigens.
Figure 2 shows the increased binding of a Fe(II) ions-treated immunoglobulin preparation for intravenous administration to human liver antigens.
Figure 3 IVIg treated as mentioned in the text to Figure 1 acquires increased binding activity to pure myosin.
Figure 4 represents the increased binding of a Fe(II) ions-treated mouse monoclonal antibody to self antigens (from a mouse liver extract).
Figure 5 presents data showing that the Fe(II) ions-treatment results in a potentiation of the idiotype/antiidiotype interactions between immunoglobulin molecules.

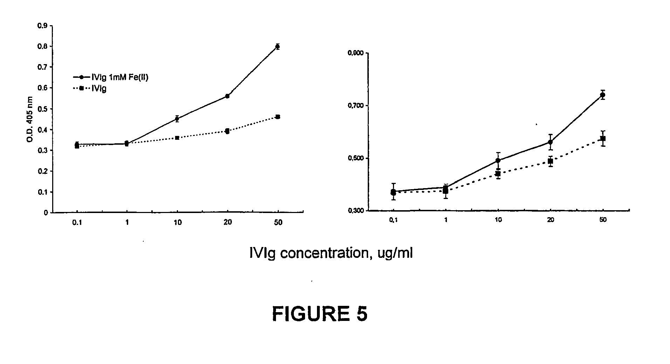

Figure 5 presents data showing that the Fe(II) ions-treatment results in a potentiation of the idiotype/antiidiotype interactions between immunoglobulin molecules.

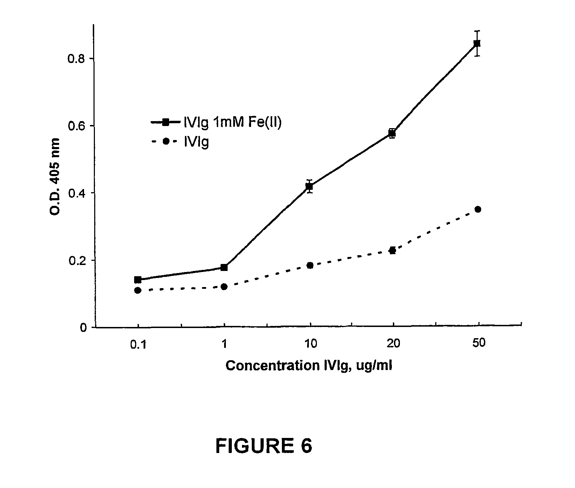

Figure 6 shows that the ferrous ions treatment of a therapeutic IVIg preparations increases the binding of the latter to the inflammatory cytokine hIFN-γ.

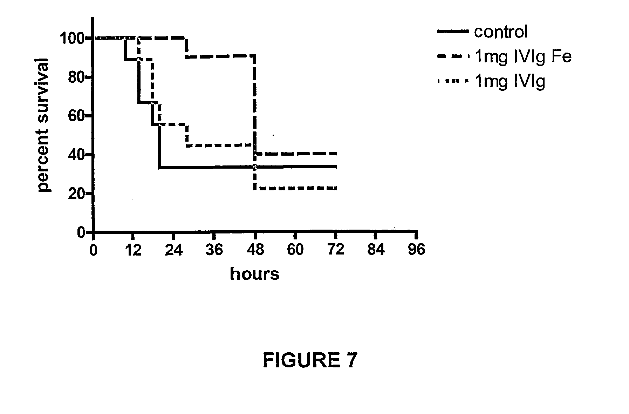

Figure 7 presents the increased survival of the mice infected with 5.10⁸ E. coli WF⁺strain and treated with Fe(II) ions-treated IVIg.

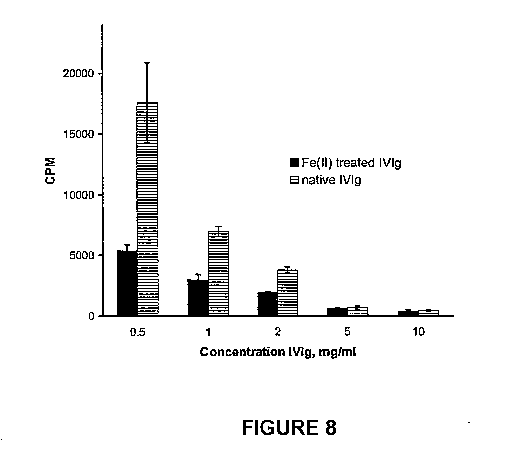

Figure 8. Effect of the ferrous ions-treated IVIg on the PHA-induced proliferation of human peripheral blood mononuclear cells.
Figure 9 shows the lack of aggregation of immunoglobulin molecules after Fe(II) ions treatment.

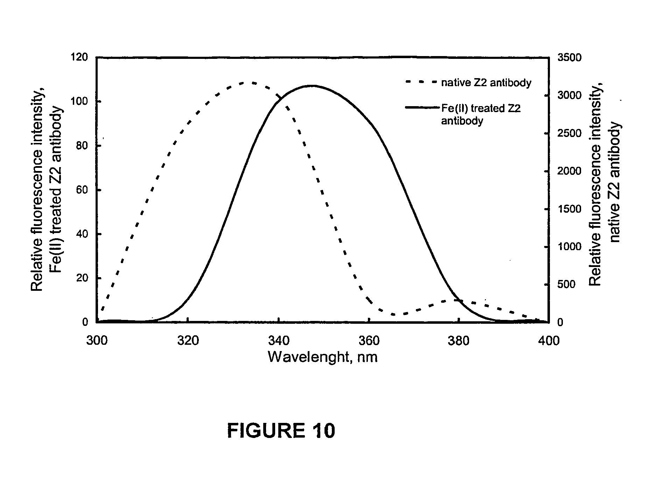

Figure 10 presents evidence that structural changes in the IgG molecules result from their contact with ferrous ions.
Figure 11 shows that superoxide anion is generated in an immunoglobulin solution by the addition of ferrous ions.

The following Examples illustrate the present invention without limiting the scope and the spirit of the same.

Example 1: Increasing of the binding activity of treated with ferrous ions immunoglobulin preparations to E.coli antigens.

Ferrous sulfate in a final concentration of 0.01mM, 0.1mM or 1mM is added to a commercial immunoglobulin preparation for intravenous administration (Intraglobin F from Biotest, Germany) and the mixture is incubated for 12 hours at neutral pH (7.4) at 4 ⁰C.

An extract of surface and periplasmic antigens of *E. coli* is subjected to SDS-PAGE electrophoresis under unreducing conditions and the separated molecules are transferred on a nitrocellulose membrane. Strips from the same membrane are incubated with the native and separately with the Fe(II) ions-treated immunoglobulin preparation, washed, incubated with an alkaline phosphatase-conjugated anti-human IgG(γ) antibody and developed. Figure 1 shows the increased binding of the Fe(II) ions treated therapeutic IVIg to the *E. coli* antigens.

Example 2. Comparative study of the autoreactivity of native- and of ferrous ions-treated immunoglobulin preparations.

Ferrous sulfate (final concentration 0.01mM, 0.1mM or 1mM) was added to Intraglobin F as described above in Example 1.

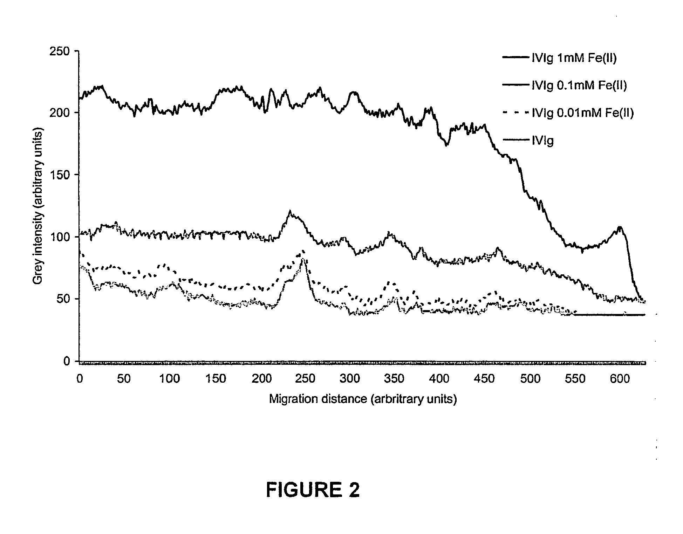

Human liver antigen extract is subjected to SDS-PAGE electrophoresis under unreducing conditions and the separated molecules transferred on a nitrocellulose membrane. Strips from the same membrane are incubated with the native and separately with the Fe(II) ions-treated immunoglobulin preparation as described in Example 1. Figure 2 shows the increased binding of the Fe(II) ions-treated IVIg to the human liver antigens that is a sign for the increased autoreactivity of the immunoglobulin preparation.

Example 3. Study of the effect of Fe(II) ions treatment of IVIg on its binding to a pure self-antigen.

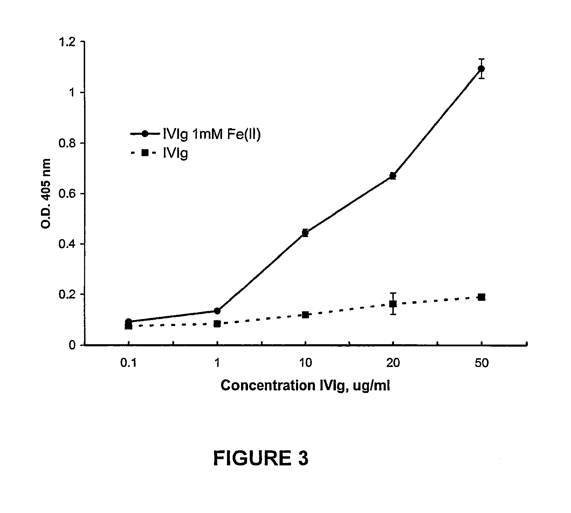

This analysis is performed using ELISA. Polystyrene ELISA plates are coated with myosin obtained from a commercial source (at 5ug/ml in PBS pH 7.4). The plates are blocked and increasing concentrations (from 0.1 to 50 ug/ml) of the native- and the treated preparations were added. The increased binding of IVIg after its contact with Fe(II) ions to the pure self-antigen is presented in Figure 3.

Example 4. Study of the effects of Fe(II) ions on a monoclonal antibody.

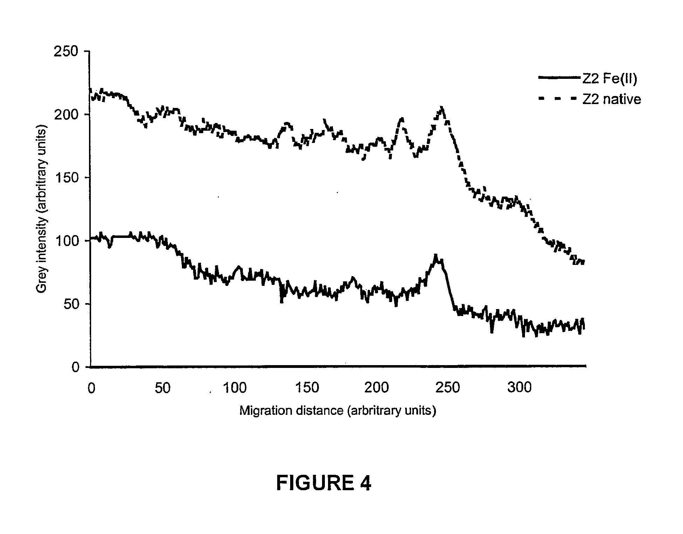

The approach described in Example 3 is used. The reactivity of the Z2 mouse monoclonal (mouse IgG2a-specific) antibody to mouse liver antigens is tested. The data show an increase in the autoreactivity (Figure 4).

Example 5. Study of the effects of ferrous ions treatment on the idiotype/antiidiotype interactions between immunoglobulin molecules.

ELISA plates are coated with pure F(ab)₂ fragments of IVIg or with pure Fc fragments of IVIg. The binding of the untreated and of the Fe(II)-treated immunoglobulin preparation to these immobilized fragments is studied by using anti human IgG (Fc-specific) or respectively with anti-human IgG (F(ab')₂- specific) alkaline phosphatase-conjugated antibodies. The data show (Figure 5) that the ferrous ions-treatment increases the binding of the therapeutic immunoglobulin preparation to the F(ab')₂ (left panel), but not to the Fcγ immunoglobulin fragments (right panel). Therefore an increase of idiotype/antiidiotype interactions takes place.

Example 6. Effect of the Fe(II) ions-treatment on the binding of natural human IgG autoantibodies to human IFN-γ.

ELISA using recombinant human IFN-γ coated plates is performed as described in text to Figure 2. Ferrous ions-treatment results in increased binding to the pro-inflammatory cytokine hIFN-γ (Figure 6).

Example 7. Effect of Fe(II)-treated IVIg on survival of mice with septic shock.

The increased ability of Fe(II)-treated IVIg preparation to bind to pro-inflammatory cytokines is confirmed by the results from *in vivo* experiments on mice with septic shock. Eight to twelve weeks-old ICR mice are injected intraperitoneally with 5.10⁸ *E. coli* (strain WF+, provided by the Central Research Institute of Experimental Medicine, Jena, Germany). Three groups with 30 animals each are formed. The mice from the first one are injected with PBS (controls), from the second one - with native IVIg and from the third - with Fe(I I) ions-treated IVIg. Groups 2 and 3 are subdivided into groups with 10 animals each that receive a single dose of 5mg, 1 mg or of 0.2 mg IVIg. The results from the study show that 24 hours after the infection 60-65% of the PBS and of the native-IVIg injected animals are dead, while all mice treated with the Fe(II) ions treated IVIg survive (Figure 7).

Example 8. Study of the ability of Fe(II)-treated immunoglobulin preparation to suppress cell proliferation.

Peripheral blood mononuclear cells were obtained from venous blood donated by a healthy volunteer. The cells are cultured *in vitro* in the presence of the mitogen PHA (16). Six hours later increasing amounts of a native and of a Fe(II)-treated IVIg preparations (at a final concentrations of 20 µg, 200 µg, 400 µg, 1000 µg or 2000 µg per milliliter) are added. The results point out that the ferrous ions-treated therapeutic pooled IgG preparation has a stronger inhibitory activity on the mitogen-induced cell proliferation (Figure 8).

Example 9. Study of the formation of molecular aggregates after the treatment of a therapeutic IVIg preparation with ferrous ions.

0.01, 0.1 and 1 mM ferrous ions are added to an immunoglobulin preparation and the latter is subjected to HPLC analysis using a TSK gel G3000 SW (7.5 mm x 60.0 cm) column and a protective TSK gel SW Guardcolumn (7.5 mm x 7.5 cm). The percentage of the immunoglobulin aggregates/dimers/monomers are presented. The comparative analysis of the chromatographic curves shows no significant additional aggregate formation after the ferrous ions-treatment of the preparation (Figure 9).

Example 10. Analysis of the structural changes of the immunoglobulin molecules after ferrous ions treatment.

A change in tryptophane fluorescence after incubation of a monoclonal antibody with 1mM Fe(II) ions is observed. The analysis is performed at room temperature using a Perkin-Elmer spectrofluorimeter at a 280 nm excitation wavelength. The emission spectrum from 300 to 400 nm is registered. A decrease in the fluorescence signal intensity and a shift in the emission maximum are detected. These are signs for structural changes in the molecule.

The shift in the emission maximum may be the result from the oxidation of tryptophan to kynurenine. This hypothesis is supported by chemiluminescence analysis using lucigenin. IVIg (10 mg/ml) is added to a buffer containing 0.5 mg/ml lucigenin. A weak chemiluminescent signal is detected. The addition of 0.5 mM ferrous ions to the same system results in the generation of a strong signal. This signal is not influenced by the addition of 10U catalase, but is suppressed in the presence of 1U superoxide dismutase. Therefore ferrous ions are responsible for the generation of superoxide anion radicals that affect the protein molecule (Figures 10 and 11).

### References

1. Coutinho, A., M. D. Kazatchkine, and S. Avrameas. 1995. Natural autoantibodies. Curr. Opin. Immunol. 7:812.
2. Notkins, A. L. 2004. Polyreactivity of antibody molecules. Trends Immunol 25:174.
3. Kazatchkine, M. D., and S. V. Kaveri. 2001. lmmunomodulation of Autoimmune and Inflammatory Diseases with Intravenous Immune Globulin. N Engl J Med 345:747.
4. Vassilev.T.L, M. D. Kazatchkine, J. -P. D. Van Huen, M. Mekrache, E. Bonnin, J. -C. Mani, C. Lecroubier, D. Korinth, D. Baruch, F. Schriever, and S. K. Kaveri. 1999. Inhibition of cell adhesion by antibodies to Arg-Gly-Asp (RGD) in normal immunoglobulin for therapeutic use (intravenous immunoglobulin, IVIg). Blood 93:3624.
5. Kaveri, S., T. Vassilev, V. Hurez, R. Lengagne, C. Lefranc, S. Cot, P. Pouletty, D. Glotz, and M. D. Kazatchkine. 1996. Antibodies to a conserved region of HLA class I molecules, capable of modulating CD8 T cell-mediated function, are present in pooled normal immunoglobulin for therapeutic use. J CHn Invest 97:865.
6. Marchalonis, J. J., H. Kaymaz, and F. e. a. Dedeoglu. 1992. Human autoantibodies reactive with synthetic autoantigens from T-cell receptor betha chain. Proc. Natl. Acad. ScL USA 89:3325.
7. Rossi, F., and M. D. Kazatchkine. 1989. Antiidiotypes against autoantibodies in pooled normal human polyspecific Ig. J. Immunol. 143:4104.
8. Rossi, F., Y. Sultan, and M. D. Kazatchkine. 1988. Anti-idiotypes against autoantibodies and alloantibodies to Factor VIII:C (anti-haemophilic factor) are present in therapeutic polyspecific normal immunoglobulins. Clin. Exp. Immunol. 74:311.
9. Kazatchkine, M. D., G. Dietrich, V. Hurez, N. Ronda, B. Bellon, F. Rossi, and S. V. Kaveri. 1994. V region-mediated selection of autoreactive repertoires by intravenous immunoglobulin (i.v.Ig). Immunol Rev 139:79.
10. Shoenfeld, Y., L. Rauova, B. Gilburd, F. Kvapil, I. Goldberg, J. Kopolovic, J. Rovensky, and M. Blank. 2002. Efficacy of IVIG affinity-purified anti-double-stranded DNA anti-idiotypic antibodies in the treatment of an experimental murine model of systemic lupus erythematosus. International Immunology 14:1303.
11. Vassilev, T. L., I. L. Bineva, G. Dietrich, S. V. Kaveri, and M. D. Kazatchkine. 1995. Variable region-connected, dimeric fraction of intravenous immunoglobulin enriched in natural autoantibodies. J AUTOIMMUN. Journal of Autoimmunity 8:405.
12. Dietrich, G., S. V. Kaveri, and M. D. Kazatchkine. 1992. A V region- connected autoreactive subfraction of normal human serum immunoglobulin G. Eur J Immunol 22:1701.
13. Jaite, J., J. Grzegorczyk, M. Zmyslony, E. Rajkowska, M. Sliwinska- Kowalska, and M. Kowalski. 2001. Influence of a 7 mT Static Magnetic and Iron Ions on Apoptosis and Necrosis in Rat Blood Lymphocytes. Journal Occup Health 43:379.
14. Klebanoff, S. J., A. M. Waltersdorph, B. R. Michel, and H. Rosen. 1989. Oxygen-based Free Radical Generation by Ferrous Ions and Deferoxamine. The Journal of Biological Chemistry 264:19765.
15. 1997. Monograph 918. In: European Pharmacopoeia. Maisonneuve, Moulins-Les-Metz, France.
16. van Schaik, I. N., I. Lundkvist, M. Vermeulen, and A. Brand. 1992. Polyvalent immunoglobulin for intravenous use interferes with cell proliferation in vitro. J CHn Immunol 12:325.

## Claims

1. Use of ferrous ions for *in vitro* increasing the immunoreactivity of immunoglobulin preparations.

2. Use of ferrous ions according to claim 1, **characterized in that** a single dose of ferrous ions is added to an immunoglobulin preparation in a final concentration from 0.5 to 2.0 Mm.

3. Use of an immunoglobulin preparation with increased immunoreactivity obtainable according to any of Claims 1 or 2 for the manufacture of a medicament for treating septic shock.

## Patentansprüche

1. Die Verwendung von Eisen(II)-lonen für die *in vitro* Erhöhung der Immunreaktivität von Immunoglobulin-Präparaten.

2. Die Verwendung von Eisen(II)-lonen nach Anspruch 1, wobei eine einzelne Dosis von Eisen(II)-lonen in einer Endkonzentration von 0,5 bis 2,0 Mm einem lmmunoglobulin-Präparat zugegeben wird.

3. Die Verwendung von Immunoglobulin-Präparaten mit erhöhter Immunreaktivität nach Ansprüchen 1 oder 2 für die Herstellung von Arzneimitteln zur Behandlung von septischen Schocks.

## Revendications

1. L'utilisation *in vitro* d'ions ferreux (Fe(II)) pour augmenter l'immunoréactivité de préparations d'immunoglobulines.

2. L'utilisation d'ions ferreux (Fe(II)) selon la revendication 1, **caractérisée par** l'addition d'une simple dose d'ions ferreux à une préparation d'immunoglobulines à une concentration finale de 0.5 à 2.0 mM.

3. L'utilisation d'une préparation d'immunoglobulines dont l'immunorécativité est augmentée, obtenue selon les revendications 1 et 2 pour la production d'un médicament destiné à traiter le choc septique.
